# EUROPEAN PATENT APPLICATION

(11) **EP 4 220 119 A1**
(43) Date of publication of application: **02.08.2023**
(21) Application number: 22215394.2
(22) Date of filing: 21.12.2022
(51) Int. Cl.: G01N 1/08

(54) **BIOLOGICAL NUCLEIC ACID COLLECTION METHOD AND BIOLOGICAL NUCLEIC ACID COLLECTION APPARATUS**

(30) Priority: 19.01.2022 JP 2022006286
(71) Applicant: Kabushiki Kaisha Toyota Chuo Kenkyusho, Nagakute-shi, Aichi-ken 480-1192 (JP)
(72) Inventor: ONO, Manami, Nagakute-shi, 480-1192 (JP); TANAKA, Hidenori, Nagakute-shi, 480-1192 (JP)
(74) Representative: Kramer Barske Schmidtchen Patentanwälte PartG mbB

(57) **Abstract**

There is provided a biological nucleic acid collection method of collecting biological nucleic acids from an environment. The biological nucleic acid collection method comprises: setting a collection target area that is a subject for collecting the biological nucleic acids in the environment; rotating a rotation body with a surface of the rotation body coming into contact with surface of ground in the collection target area, so as to cause nucleic acids-containing sediment including the biological nucleic acids present on the surface of ground to adhere to the surface of the rotation body; and collecting the nucleic acids-containing sediment from the rotation body.

## Description

### Technical Field

The present disclosure relates to a biological nucleic acid collection method and a biological nucleic acid collection apparatus.

### Background

One method of surveying or monitoring species of organisms present in an environment is a method of collecting biological nucleic acids from the environment and analyzing the collected nucleic acids to identify the specifies of organisms which the nucleic acids are derived from. The biological nucleic acids are nucleic acids that include genetic information of organisms and that are released from the organisms into the environment, and are included in nucleic acids present in the environment, such as environmental DNAs. A known method of collecting such biological nucleic acids from the environment and analyzing the collected biological nucleic acids analyzes water samples collected from water areas such as rivers (environmental water samples) by environmental DNA analysis (as described in, for example, Patent Literature 1 and Patent Literature 2). A known method of detecting terrestrial mammal species collects the soil in a neighborhood of a fixed point camera (for example, by sampling the soil of 2 cm in depth at every 2 meter intervals) and performs environmental DNA analysis in combination with the results of image analysis of the camera (as described in, for example, Non-Patent Literature 1).
Patent Literature 1: JP 2017-99376A
Patent Literature 2: JP 2020-92645A

Non-Patent Literature 1: Kevin Leempoel et al. A comparison of eDNA to camera trapping for assessment of terrestrial mammal diversity, Proceedings of the Royal Society B., 2020, Jan. 15, vol., 287 (1918)

### Summary

### Technical Problem

The method of analyzing the water samples, however, only collects nucleic acids of fish and waterfowl living in the water areas which the samples are collected from or nucleic acids of animals visiting the water areas. There is accordingly a demand for collecting and analyzing the biological nucleic acids of terrestrial organisms living away from the water areas. Particularly, the nucleic acids are readily decomposed in water. Collection of biological nucleic acids from the land where the biological nucleic acids are present in a dry state that are relatively hardly decomposed is expected to allow for exhaustive survey and monitoring of the species of living organisms. Collection of the soil as the object of analysis for collection of biological nucleic acids in the land areas as described in Non-Patent Literature 1 has a difficulty in sampling in a wide range. Accordingly, there is a demand for the technique of collecting nucleic acids derived from terrestrial organisms in a wide range of areas.

### Solution to Problem

The present disclosure may be implemented by aspects described below.
(1) According to one aspect of the present disclosure, there is provided a biological nucleic acid collection method of collecting biological nucleic acids from an environment. This biological nucleic acid collection method comprises: setting a collection target area that is a subject for collecting the biological nucleic acids in the environment; rotating a rotation body with a surface of the rotation body coming into contact with surface of ground in the collection target area, so as to cause nucleic acids-containing sediment including the biological nucleic acids present on the surface of ground to adhere to the surface of the rotation body; and collecting the nucleic acids-containing sediment from the rotation body.
   The biological nucleic acid collection method of this aspect rotates the rotation body provided in a biological nucleic acid collection apparatus with the surface of the rotation body coming into contact with the surface of ground in the set collection target area, so as to cause the nucleic acids-containing sediment including the biological nucleic acids present on the surface of ground to adhere to the surface of the rotation body, and collects the nucleic acids-containing sediment. The configuration of this aspect requires only a simple operation of rotating the rotation body in the state that the rotation body comes into contact with the surface of ground, with a view to collecting the nucleic acids-containing sediment on the surface of ground. This configuration accordingly enables the nucleic acids derived from terrestrial organisms to be sampled exhaustively from a wide range in the collection target area. The nucleic acids-containing sediment collected from the surface of ground may include not only nucleic acids derived from organisms living on the surface of ground but biological nucleic acids released in the atmosphere. This accordingly allows for collection of biological nucleic acids in a wider range of species of organisms which live or stayed in the collection target area.
(2) The biological nucleic acid collection method of the above aspect may further comprise extracting nucleic acids from the collected nucleic acids-containing sediment. The configuration of this aspect allows for extraction of nucleic acids in a wider range of species of organisms with suppressing a deviation, with regard to organisms that live or stayed in the collection target area.
(3) According to another aspect, there is provided a biological nucleic acid collection apparatus configured to collect biological nucleic acids from an environment. The biological nucleic acid collection apparatus comprises a rotation body configured to rotate accompanied with moving of the biological nucleic acid collection apparatus while coming into contact with surface of ground, so as to cause nucleic acids-containing sediment including biological nucleic acids present on the surface of ground to adhere to a surface of the rotation body; and a collector configured to collect the nucleic acids-containing sediment adhering to the surface of the rotation body.

The biological nucleic acid collection apparatus of this aspect rotates the rotation body provided in the biological nucleic acid collection apparatus in the state that the surface of the rotation body comes into contact with the surface of ground, so as to cause the nucleic acids-containing sediment including the biological nucleic acids present on the surface of ground to adhere to the surface of the rotation body, and subsequently uses the collector to collect the nucleic acids-containing sediment adhering to the surface of the rotation body. The configuration of this aspect requires only a simple operation of rotating the rotation body in the state that the rotation body comes into contact with the surface of ground, with a view to collecting the nucleic acids-containing sediment on the surface of ground. This configuration accordingly enables the nucleic acids derived from terrestrial organisms to be sampled exhaustively from a wide range. The nucleic acids-containing sediment collected from the surface of ground may include not only nucleic acids derived from organisms living on the surface of ground but biological nucleic acids released in the atmosphere. This accordingly allows for collection of biological nucleic acids in a wider range of species of organisms.

(4) In the biological nucleic acid collection apparatus of the above aspect, the collector may come into contact with the surface of the rotation body to collect the nucleic acids-containing sediment adhering to the surface of the rotation body. The configuration of this aspect enables the nucleic acids-containing sediment to be collected by simply bringing the collector into contact with the surface of the rotation body.

(5) In the biological nucleic acid collection apparatus of the above aspect, the collector may comprise at least one of a rotational member, a brush-like member and a duster-like member. The configuration of this aspect efficiently performs the operation of collecting the nucleic acids-containing sediment adhering to the surface of the rotation body by using the collector.

(6) In the biological nucleic acid collection apparatus of the above aspect, the collector may be provided to be attached to and detached from the rotation body, such as to form the surface of the rotation body that comes into contact with the surface of ground. The configuration of this aspect enables the nucleic acids-containing sediment to be collected by detaching the collector that forms the surface of the rotation body after adhesion of the nucleic acids-containing sediment to the surface of the rotation body.

(7) In the biological nucleic acid collection apparatus of the above aspect, the collector may be formed in a sheet-like shape. The configuration of this aspect facilitates the formation of the surface of the rotation body by the collector, as well as the attachment and the detachment of the collector to and from the rotation body. This accordingly enhances the efficiency of collection of the biological nucleic acids by using the collector.

(8) In the biological nucleic acid collection apparatus of the above aspect, the collector may be made of at least one of a rubber, a resin, and a non-woven fabric. The configuration of this aspect enables the nucleic acids-containing sediment present on the surface of ground to efficiently adhere to the collector.

(9) In the biological nucleic acid collection apparatus of the above aspect, a ratio of an area of the rotation body to a total area of the entire biological nucleic acid collection apparatus that is viewed in projection in a vertical direction may be not less than 8%. The configuration of this aspect ensures the sufficient area of the rotation body and thereby enhances the efficiency of collection of the biological nucleic acids by using the rotation body.

(10) The biological nucleic acid collection apparatus of the above aspect may further comprise a connection member configured to connect the rotation body with an external running vehicle configured to autonomously move. The configuration of this aspect enables the nucleic acids-containing sediment to be collected by the rotation body provided as a separate body from the running vehicle configured to autonomously move.

The preset disclosure may be implemented by a variety of aspects other than those described above, for example, a method of extracting biological nucleic acids and a method of analyzing biological nucleic acids.

### Brief Description of Drawings

Fig. 1 is a flowchart showing a method of collecting biological nucleic acids;
Fig. 2 is explanatory diagrams illustrating the state of collecting biological nucleic acids by using an apparatus for collecting biological nucleic acids;
Fig. 3 is an explanatory diagram illustrating an operation of collecting the biological nucleic acids in the apparatus for collecting biological nucleic acids;
Fig. 4 is a flowchart showing one example of a method of extracting the biological nucleic acids;
Fig. 5 is a flowchart showing another example of the method of extracting the biological nucleic acids;
Fig. 6 is explanatory diagrams illustrating the state of collecting biological nucleic acids by using another apparatus for collecting biological nucleic acids;
Fig. 7 is explanatory diagrams illustrating the state of collecting biological nucleic acids by using another apparatus for collecting biological nucleic acids; and
Fig. 8 is an explanatory diagram illustrating results of analysis of environmental DNA samples.

### Description of Embodiments

### A. First Embodiment

### (A-1) Outline of Method of Collecting Biological Nucleic Acids

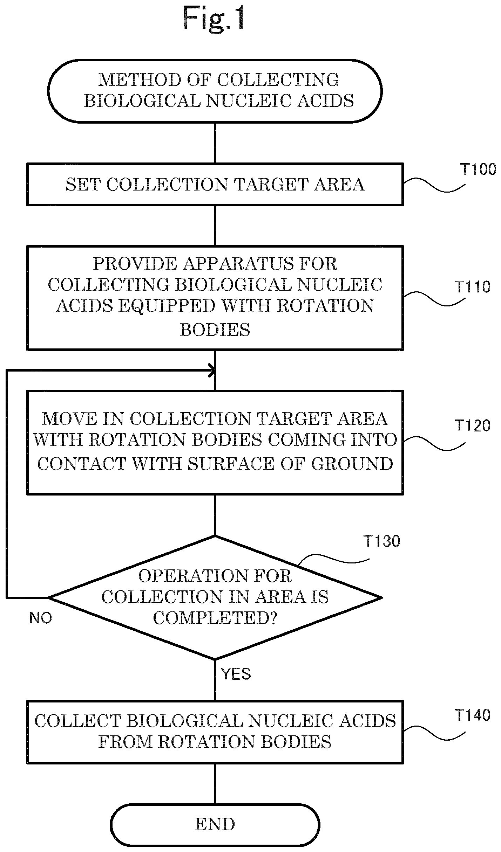

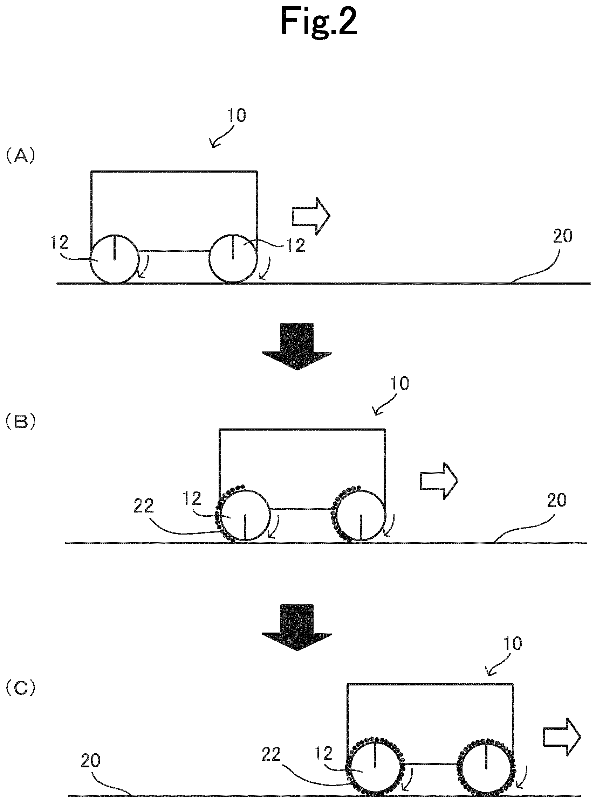

Fig. 1 is a flowchart showing a biological nucleic acid collection method or a method of collecting biological nucleic acids according to a first embodiment. Fig. 2 is explanatory diagrams schematically illustrating the state of performing part of the method of collecting biological nucleic acids shown in Fig. 1 by using a biological nucleic acid collection apparatus or an apparatus for collecting biological nucleic acids 10. The biological nucleic acids herein denote substances constituting organisms as nucleic acids including genetic information of the organisms. The biological nucleic acids to be collected are deoxyribonucleic acids (DNAs). Biological nucleic acids including ribonucleic acids (RNAs) may, however, be an object to be collected and analyzed. According to the embodiment, with a view to collecting the biological nucleic acids, a nucleic acids-containing sediment including the biological nucleic acids is collected from the surface of ground by using the apparatus for collecting biological nucleic acids 10. The nucleic acids-containing sediment includes cells and pieces of tissue separated from a variety of organisms, substances derived from excrement, and substances derived from dead organisms (fragments and the like). The nucleic acids-containing sediment present on the surface of ground includes biological nucleic acids that are derived from a variety of species of organisms present on or above the surface of ground (for example, on the surface of ground, above the ground, in the air). The following describes the outline of the method of collecting biological nucleic acids with reference to Fig. 1 and Fig. 2.

In the process of collecting the biological nucleic acids, an examiner performing survey and monitoring of the species of organisms first sets a collection target area that is a subject for collection of biological nucleic acids (process T 100). The collection target area denotes an area that is a subject for survey and monitoring of the species of organisms living therein. Collecting and analyzing the biological nucleic acids from the set collection target area allows for survey and monitoring of the species of organisms living in the area.

The examiner subsequently provides an apparatus for collecting biological nucleic acids (process T110). As shown in Fig. 2(A), the apparatus for collecting biological nucleic acids 10 according to the embodiment is a land moving vehicle provided with rotation bodies 12 that are wheels for running. The configuration of the apparatus for collecting biological nucleic acids 10 will be described in detail later. In Fig. 2(A) to 2(C), a direction of moving the apparatus for collecting biological nucleic acids 10 is indicated by open arrows.

The examiner moves the apparatus for collecting biological nucleic acids 10 with bringing the rotation bodies 12 thereof into contact with the surface of ground in the collection target area set at the process T 100 (process T120). As shown in Fig. 2(B) and Fig. 2(C), in the process of moving the apparatus for collecting biological nucleic acids 10, the rotation bodies 12 rotate and move with coming into contact with surface of ground 20, accompanied with the move of the apparatus for collecting biological nucleic acids 10. As a result, the rotation bodies 12 are pressed against the surface of ground 20 and thereby causes a nucleic acids-containing sediment 22 present on the surface of ground 20 to adhere to the surface of the rotation bodies 12. A location where the apparatus for collecting biological nucleic acids 10 moves in the collection target area is preferably a location having the flat surface of ground 20 and having a larger contact area between the rotation bodies 12 and the surface of ground 20, for example, a paved road. The apparatus for collecting biological nucleic acids 10 may, however, run on any of various surfaces of ground, for example, a soilexposed surface of ground or a lawn-covered or another plant-covered surface of ground. The adhesion of the nucleic acids-containing sediment to the rotation bodies 12 is affected by, for example, the weather, as well as the state of the surface of ground described above. More specifically, after the rainy weather, the nucleic acids-containing sediment on the surface of ground may be flowed away to disappear, or different species of organisms different from those in the clear weather may appear on the surface of ground, so that the state of the nucleic acids-containing sediment may be changed. In the case of repeated monitoring of the species of organisms in the collection target area by analyzing the collected biological nucleic acids, it is desirable to equalize the conditions for collection of the nucleic acids-containing sediment in the collection target area.

After the start of the operation of causing the nucleic acids-containing sediment to adhere to the surface of the rotation bodies 12 at the process T120, when it is determined at process T130 that the operation for collection of the biological nucleic acids in the collection target area has not yet been completed (process T130: NO), the examiner continues the operation of the process T120. One procedure may determine that the operation for collection of the biological nucleic acids has been completed, for example, when a moving distance of the apparatus for collecting biological nucleic acids 10 moving in the collection target area reaches a predetermined reference value or when a moving time of the apparatus for collecting biological nucleic acids 10 reaches a predetermined reference value. Another procedure may set in advance a moving pattern of the apparatus for collecting biological nucleic acids 10 in the collection target area and may, when the move according to this pattern is terminated, determine that the operation for collection of the biological nucleic acids has been completed. Increasing the moving distance of the apparatus for collecting biological nucleic acids 10 to increase the opportunity of the rotation bodies 12 coming into contact with the surface of ground 20 increases the amount of the biological nucleic acids adhering to the rotation bodies 12.

When it is determined at the process T130 that the operation for collection of the biological nucleic acids in the collection target area has been completed (process T130) YES), the examiner collects the biological nucleic acids from the rotation bodies 12 (process T140). The operation of collecting the nucleic acids-containing sediment from the rotation bodies 12 and obtaining the biological nucleic acids from the nucleic acids-containing sediment will be described later in detail.

### (A-2) Configuration of Apparatus for Collecting Biological Nucleic Acids

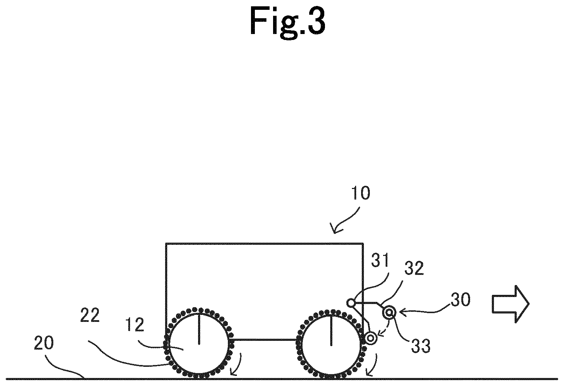

Fig. 3 is an explanatory diagram illustrating the operation of collecting the biological nucleic acids adhering to the rotation bodies 12 provided in the apparatus for collecting biological nucleic acids 10 of the first embodiment. As shown in Fig. 3, the apparatus for collecting biological nucleic acids 10 is provided with collectors 30 in addition to the rotation bodies 12 as a configuration involved in collection of the biological nucleic acids. The collector 30 is omitted from the illustration of Fig. 2 described above. The following sequentially describes the general configuration of the apparatus for collecting biological nucleic acids 10 and the configuration involved in collection of the biological nucleic acids using the rotation bodies 12 and the collectors 30.

As described above, the apparatus for collecting biological nucleic acids 10 used in the method of collecting biological nucleic acids according to the embodiment is the land moving vehicle provided with the rotation bodies 12 that are wheels for running. The apparatus for collecting biological nucleic acids 10 is illustrated in Fig. 2 as a four-wheeled vehicle that is provided with four wheels serving as the rotation bodies 12, but may have a different configuration, such as a three-wheeled vehicle, a two-wheeled vehicle or a single-wheeled vehicle. The apparatus for collecting biological nucleic acids 10 may be a self-propelled moving vehicle with a power source, such as an electric motor or an internal combustion engine, mounted thereon to move the apparatus for collecting biological nucleic acids 10 or may be a non-self-propelled moving vehicle that is made movable by externally applying a power, such as human power. In the description below, the self-propelled moving vehicle and the non-self-propelled moving vehicle are collectively referred to as the "moving vehicle".

The apparatus for collecting biological nucleic acids 10 may be a manned apparatus with a driver riding thereon to perform an operation relating to moving or may be an unmanned apparatus configured to perform an automatic operation or a remote operation. The unmanned apparatus configured to perform the automatic operation or the remote operation allows for further reduction in the size of the entire apparatus for collecting biological nucleic acids 10. The unmanned apparatus is thus enabled to enter a narrow location where the manned apparatus has difficulty in entering, and to collect the biological nucleic acids. The apparatus for collecting biological nucleic acids 10 may be configured as an exclusive apparatus for collecting biological nucleic acids or may have a basic structure that is a moving vehicle, such as a vehicle for general use or a self-propelled vehicle for any of various uses including agriculture use and building use.

With regard to the rotation bodies 12 of the apparatus for collecting biological nucleic acids 10, an increase in the contact area of the rotation bodies 12 that is in contact with the surface of ground 20 increases the amount of collection of nucleic acids per operation of collecting the biological nucleic acids shown in Fig. 1, i.e., an amount of information obtainable as nucleic acids. Accordingly, in terms of increasing the amount of collection of the nucleic acids, the ratio of the area of the rotation bodies 12 to the total area of the apparatus for collecting biological nucleic acids 10 that is viewed in projection in a vertical direction is not less than 1%, is preferably not less than 8%, is more preferably not less than 10% and is furthermore preferably not less than 20%. Especially when the apparatus for collecting biological nucleic acids 10 is configured as an exclusive apparatus for collecting biological nucleic acids, it is easy to increase the area of the rotation bodies 12 relative to the total area of the apparatus for collecting biological nucleic acids 10.

The rotation bodies 12 may be made of any material that has substantially no problem in use as the wheels for running of the apparatus for collecting biological nucleic acids 10 and that enables the nucleic acids-containing sediment to adhere to when being pressed against the surface of ground 20. In the case where the rotation bodies 12 are reused for collection of the biological nucleic acids, the rotation bodies 12 may be made of a material that allows for repetition of processes for suppressing the biological nucleic acids adhering to the rotation bodies 12 in a previous use from being left or more specifically a process of washing the rotation bodies 12 and a nucleic acid removal process using a chlorine bleaching agent, a nucleolytic agent or the like. In the rotation bodies 12, for example, a base portion thereof may be made of stainless steel or a silicone resin and at least a surface portion thereof that comes into contact with the surface of ground may be made of a rubber or a resin.

The collector 30 shown in Fig. 3 is a member configured to come into contact with the surface of the rotation body 12 and to collect the nucleic acids-containing sediment adhering to the surface of the rotation body 12. The collector 30 is provided near the rotation body 12, and is kept at a position separate from the surface of the rotation body 12 while the apparatus for collecting biological nucleic acids 10 is moved to perform an operation of causing the nucleic acids-containing sediment to adhere to the rotation body 12. After termination of the operation of causing the nucleic acids-containing sediment to adhere to the rotation body 12, the collector 30 is moved to be pressed against the surface of the rotation body 12 at the process T 140 shown in Fig. 1.

In the apparatus for collecting biological nucleic acids 10 shown in Fig. 3, the collector 30 is provided at an edge of an arm 32 that is rotated to any arbitrary position about a rotating shaft 31 provided in the apparatus for collecting biological nucleic acids 10. For example, the arm 32 is rotated by a predetermined angle by using a motor or the like to press the collector 30 against the rotation body 12 and to keep the collector 30 at the position. According to a modified configuration, the collector 30 may be fixed at an edge of a chain member to be suspended above the rotation body 12. In this configuration, for example, the member of suspending the collector 30 may be moved in the vertical direction to bring the collector 30 into contact with an upper portion of the rotation body 12 and to press the collector 30 against the rotation body 12 by the dead weight of the collector 30. When the apparatus for collecting biological nucleic acids 10 is further moved in the state that the collector 30 is pressed against the rotation body 12 as described above, the rotation of the rotation body 12 causes the nucleic acids-containing sediment adhering to the surface of the rotation body 12 to be collected by the collector 30.

The collector 30 may have any configuration that comes into contact with the rotation body 12 and that enables the nucleic acids-containing sediment adhering to the surface of the rotation body 12 to be collected. Increasing the surface area of the collector 30 that comes into contact with the rotation body 12 increases the amount of the nucleic acids-containing sediment collected from the rotation body 12 by the collector 30. In terms of providing a sufficient surface area that comes into contact with the rotation body 12, the collector 30 may be configured by, for example, a rotatable member. Fig. 3 illustrates the configuration of the collector 30 by the rotatable member. The collector 30 as the rotatable member shown in Fig. 3 is formed in a columnar shape and has a rotating shaft 33 that is provided at the edge of the arm 32 to overlap with a center axis of the collector 30 in the columnar shape. When a side face of the collector 30 in the columnar shape is pressed against the rotation body 12, the collector 30 is rotated along with rotation of the rotation body 12 to cause the nucleic acids-containing sediment to be transferred and collected from the surface of the rotation body 12 to the collector 30. Using the collector 30 having the rotatable shape enables the nucleic acids-containing sediment to be collected with high efficiency.

In another example, the collector 30 may be configured by a brush-like member. The collector 30 as the brush-like member has hair ends thereof that are pressed against the rotation body 12 and thereby enables the nucleic acids-containing sediment to be collected with high efficiency. In another example, the collector 30 may be configured by a duster-like member or more specifically a member formed by bundling a plurality of long sheet members at base ends thereof. The long sheet members may be made of a cloth, a resin, or a rubber. This configuration increases the total area of the members forming the collector 30 and enables the nucleic acids-containing sediment to be collected by the collector 30 with high efficiency. The collector 30 may, however, have a different configuration from any of these configurations described above and may have any configuration that comes into contact with the surface of the rotation body 12 and that enables the nucleic acids-containing sediment adhering to the surface of the rotation body 12 to be collected.

In the process of rotating the rotation body 12 with the collector 30 pressed against the rotation body 12, increasing the moving distance of the apparatus for collecting biological nucleic acids 10 along with the rotation of the rotation body 12 increases the amount of the nucleic acids-containing sediment collected by the collector 30. Fig. 3 illustrates the collector 30 placed for one rotation body 12 among the plurality of rotation bodies 12. The collector 30 is provided for all the wheels used as the rotation bodies 12 for collection of the nucleic acids-containing sediment. A plurality of the collectors 30 may be provided for one rotation body 12.

### (A-3) Extraction and Analysis of Biological Nucleic Acids

The following describes an operation of extracting the biological nucleic acids from the nucleic acids-containing sediment collected by the collector 30 and the outline of analysis of the biological nucleic acids.

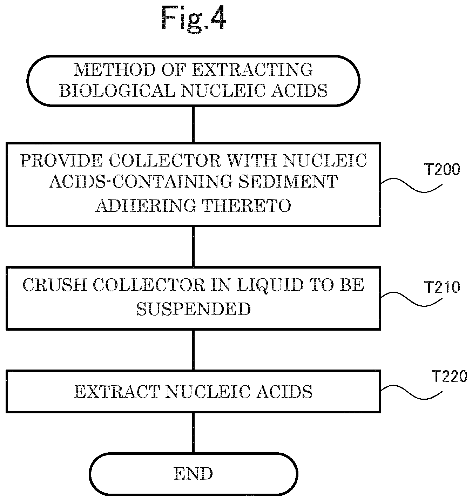

Fig. 4 is a flowchart showing one example of a method of extracting the biological nucleic acids. Fig. 4 illustrates an example of using a relatively small collector 30 and directly extracting the nucleic acids from the collector 30. The method of extracting the biological nucleic acids first provides the collector 30 with the nucleic acids-containing sediment adhering thereto (process T200). This collector 30 is provided to collect the nucleic acids-containing sediment from the rotation body 12 at the process T140 in Fig. 1.

The method subsequently crushes the collector 30 in a liquid to be suspended (process T210). The liquid used here may be a lysis buffer that lyses cells and tissues. The collector 30 may be crushed by adding, for example, ceramic beads with the collector 30 in the liquid and stirring the liquid. Crushing the collector 30 in this manner causes the nucleic acids-containing sediment adhering to the collector 30 to be separated from the collector 30 and causes the biological nucleic acids included in the nucleic acids-containing sediment to be eluted into the suspension of the crushed collector 30.

The method subsequently extracts the biological nucleic acids from the suspension with the biological nucleic acids eluted therein (process T220). For example, a procedure of extracting the biological nucleic acids centrifuges the suspension, collects a liquid moiety with the biological nucleic acids lysed therein as a supernatant liquid, removes impurity by selecting an appropriate column, and uses a nucleic acid-adsorbing column to purify the biological nucleic acids. The impurity may be, for example, a substance interfering with a reaction included in a PCR process described later. The process of extracting the nucleic acids shown as the processes T210 and T220 is a known method and may use, for example, a commercially available soil DNA extraction kit.

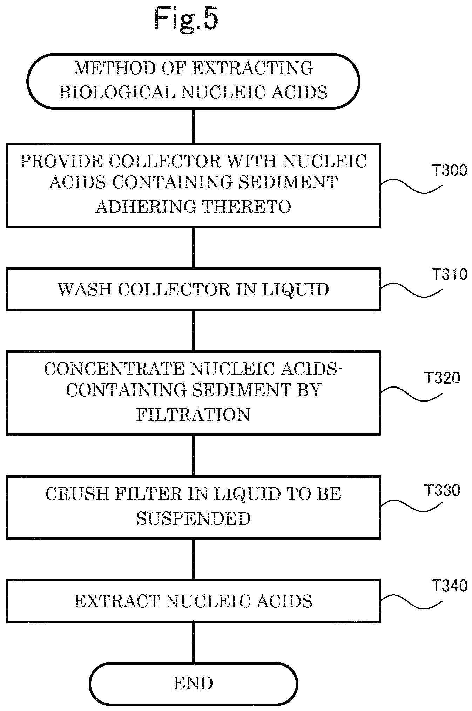

Fig. 5 is a flowchart showing another example of the method of extracting the biological nucleic acids. Fig. 5 illustrates an example of using a relatively large collector 30 and performing an operation of concentrating the nucleic acids-containing sediment adhering to the collector 30 prior to extraction of nucleic acids. The method of extracting the biological nucleic acids first provides the collector 30 with the nucleic acids-containing sediment adhering thereto (process T300), like the process T200.

The method subsequently washes the provided collector 30 in a liquid (process T310). The liquid used here may be a nuclease-free buffer or nuclease-free water that does not contain nuclease or nucleolytic enzyme. Washing the collector 30 in such a liquid causes the nucleic acids-containing sediment to be separated from the collector 30 and transferred into the liquid to be suspended. The method subsequently filtrates the suspension including the nucleic acids-containing sediment and obtains a residue including the nucleic acids-containing sediment, so as to concentrate the nucleic acids-containing sediment on a filter (process T320). The filter used here has a pore diameter that is appropriately selected to efficiently collect the nucleic acids-containing sediment included in the substances adhering to the collector 30. The pore diameter of the filter used for concentrating the nucleic acids-containing sediment is, for example, preferably 0.22 to 10 µm and is more preferably 0.22 to 5 µm. In the filtration process, the efficiency of filtration may be enhanced, for example, by using a vacuum pump to generate a negative pressure or by using a pump to apply a pressure.

The substances adhering to the collector 30 may include unrequired substances that are not readily usable as the extraction subject of the biological nucleic acids. In order to enhance the efficiency of the concentration of the nucleic acids-containing sediment by the filtration described above and the efficiency of the subsequent extraction of nucleic acids, such unrequired substances may be removed, prior to the filtration for the concentration of the nucleic acids-containing sediment. For the purpose of removal of the unrequired substances, prior to the concentration of the nucleic acids-containing sediment, the suspension may be filtrated by using a filter of a larger pore diameter (for example, a pore diameter of approximately 100 µm) than the pore diameter of the filter used for the concentration of the nucleic acids-containing sediment described above. The filtrate obtained is then subjected to the filtration for the concentration of the nucleic acids-containing sediment. The unrequired substances described above include, for example, relatively large fragments of plants, such as leaves, dead organisms such as dead insects, and stones.

After obtaining the filter with the concentrated nucleic acids-containing sediment at the process T320, the method crushes the filter in a liquid to be suspended (process T330). This process T330 is a similar process to the process T210 of Fig. 4. This causes the nucleic acids-containing sediment concentrated on the filter to be separated from the filter and causes the biological nucleic acids included in the nucleic acids-containing sediment to be eluted into the suspension of the crushed filter. The method subsequently extracts the biological nucleic acids from the suspension with the biological nucleic acids eluted therein (process T340). The process T340 is a similar process to the process T220 of Fig. 4.

The biological nucleic acids extracted as described above may be analyzed by any of various known methods. For example, the biological nucleic acids may be subjected to species-specific analysis, for example, the presence or the absence of a specific species of organism or determination of abundance by quantitative PCR (qPCR). Analysis narrowed to a specific group of species, for example, bird species or mammal species, may be allowed by performing PCR with primers appropriately selected for the specific group of species. A wider range of species may be identified simultaneously by performing PCR with appropriately selected primers. Exhaustive analysis for identification of the species of organisms or haplotype analysis in an identical species may be performed by matching of base sequences obtained by using a next generation sequencer or the like with database. Such analyses give information of the organisms living or staying in the collection target area.

The above configuration of the method of collecting biological nucleic acids and the apparatus for collecting biological nucleic acids 10 according to the embodiment causes the surface of the rotation body 12 provided in the apparatus for collecting biological nucleic acids 10 to come into contact with the surface of ground 20 and to be rotated in the set collection target area and thereby causes the nucleic acids-containing sediment 22 including the biological nucleic acids present on the surface of ground 20 to adhere to the surface of the rotation body 12. The configuration subsequently causes the collector 30 to come into contact with the surface of the rotation body 12 and thereby collect the nucleic acids-containing sediment 22 adhering to the rotation body 12. This configuration collects the nucleic acids-containing sediment on the surface of ground by simply causing the rotation body to come into contact with the surface of ground and to be rotated. This accordingly enables the nucleic acids derived from terrestrial organisms to be sampled exhaustively from a wider range in the collection target area. More specifically, the configuration of collecting the nucleic acids-containing sediment by using the rotation body 12 reduces the deviation of locations where the samples are actually collected in the collection target area. For example, when the soil is a collection object, the locations of collecting samples are generally dispersed in the collection target area. The configuration of using the rotation body 12 to collect the nucleic acids-containing sediment 22 like the above embodiment allows for sampling from a continually spreading wider range in the collection target area. This accordingly allows for exhaustive analyses including survey and monitoring of the species of organisms living in the collection target area.

The nucleic acids-containing sediment collected from the surface of ground 20 according to the embodiment may include not only nucleic acids derived from organisms living on the surface of ground but biological nucleic acids released in the atmosphere. This is because substances that include biological nucleic acids and that have a certain degree of weight, such as tissues or excrement of the organisms, are accumulated on the surface of ground. The configuration of the embodiment accordingly allows for analysis of various species of organisms present on or above the surface of ground. For example, in the case of sampling from the soil, the biological nucleic acids obtained are more likely to be restricted to the nucleic acids derived from the organisms living in the sampled soil. The configuration of the embodiment, however, allows for collection of the biological nucleic acids in a wider range of species of organisms living or staying in the collection target area. Especially, the configuration of the embodiment samples the nucleic acids-containing sediment from the dry environment in the land areas. Compared with sampling from the water areas, sampling from the environment where the nucleic acids are stably present enhances the efficiency of the collection of the biological nucleic acids.

In the first embodiment described above, the apparatus for collecting biological nucleic acids 10 is provided with the collectors 30 in addition to the rotation bodies 12. The method of collecting biological nucleic acids 10 may, however, be performed by a different configuration. More specifically, a moving vehicle without the collectors 30 may be used as the apparatus for collecting biological nucleic acids. The process T140 of Fig. 1 may be performed by using a collection member provided as a separate member from the moving vehicle and causing the nucleic acids-containing sediment 22 adhering to rotation bodies 12 provided in this moving vehicle to be collected, for example, manually from the rotation bodies 12 of the moving vehicle to the collection member. For example, the rotatable member, the brush-like member or the duster-like member described as the examples of the collector 30 provided in the apparatus for collecting biological nucleic acids 10 of the first embodiment may be used as the collection member provided to manually collect the nucleic acids-containing sediment 22 from the rotation bodies 12. The collection member may also be a swab-like member and may have any configuration that enables the nucleic acids-containing sediment to be collected from the surface of the rotation bodies 12.

### B. Second Embodiment

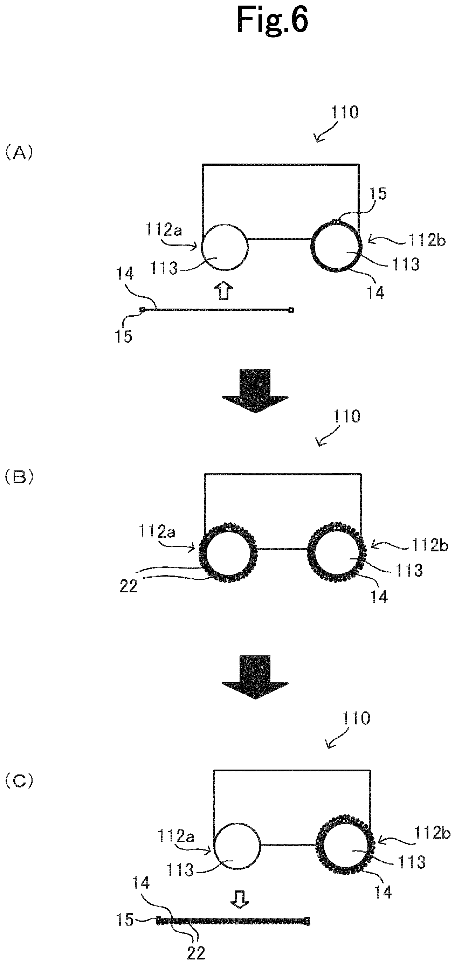

Fig. 6 is explanatory diagrams schematically illustrating the state of performing the method of collecting biological nucleic acids shown in Fig. 1 by using an apparatus for collecting biological nucleic acids 110 according to a second embodiment. The following describes an operation of collecting the biological nucleic acids by using the apparatus for collecting biological nucleic acids 110 with reference to Fig. 6 and Fig. 1.

The apparatus for collecting biological nucleic acids 110 of the second embodiment provided at the process T110 of Fig. 1 has a similar configuration to that of the apparatus for collecting biological nucleic acids 10 of the first embodiment, except that the apparatus for collecting biological nucleic acids 110 is provided with rotation bodies 112 in place of the rotation bodies 12 and with collectors 14 included in the rotation bodies 112 in place of the collectors 30. In the description of the second embodiment, the rotation bodies provided in the apparatus for collecting biological nucleic acids 110 are collectively referred to as the rotation body or the rotation bodies 112 when no distinction is needed but are individually referred to as rotation bodies 112a and 112b when distinction is needed.

The apparatus for collecting biological nucleic acids 110 is a land moving vehicle provided with the rotation bodies 122 serving as wheels for running, which rotate accompanied with moving of the apparatus for collecting biological nucleic acids 110, while coming into contact with the surface of ground. The apparatus for collecting biological nucleic acids 110 may be a four-wheeled vehicle as shown in Fig. 6 or may have a different configuration, such as a three-wheeled vehicle, a two-wheeled vehicle or a single-wheeled vehicle. The apparatus for collecting biological nucleic acids 110 may be a self-propelled moving vehicle or a non-self-propelled moving vehicle. The apparatus for collecting biological nucleic acids 110 may also be a manned apparatus or an unmanned apparatus.

As shown in Fig. 6(A), the rotation body 112 provided in the apparatus for collecting biological nucleic acids 110 includes a rotation body base portion 113 and a collector 14. The rotation body base portion 113 rotates on the surface of ground, accompanied with moving of the apparatus for collecting biological nucleic acids 110. The collector 14 is arranged to be attached to and detached from the rotation body base portion 113, such as to cover a surface of the rotation body base portion 113 that is opposed to the surface of ground. The collector 14 is a member that causes nucleic acids-containing sediment 22 including biological nucleic acids present on the surface of ground to adhere thereto when the collector 14 comes into contact with the surface of ground accompanied with the rotation of the rotation body base portion 113. Fig. 6(A) illustrates the state of the rotation body 112a prior to attachment of the collector 14 to the rotation body base portion 113 and the state of the rotation body 112b after attachment of the collector 14 to the rotation body base portion 113. The collector 14 may have any configuration that covers the surface of the rotation body base portion 113 which is opposed to the surface of ground. For example, as shown in Fig. 6(A), the collector 14 may be a sheet-like member configured to be wound around a side face of the rotation body base portion 113 opposed to the surface of ground. The configuration of winding the collector 14 around the entire circumference of the side face of the rotation body base portion 113 that comes into contact with the surface of ground and winding the collector 14 such as to cover the entire side face of the rotation body base portion 113 that comes into contact with the surface of ground enables the larger area of the collector 14 to be provided to collect the biological nucleic acids. The collector 14 is not limited to the sheet-like member but may be provided as, for example, a film-like member to cover the surface of the rotation body base portion 113. In another example, the collector 14 may be a cylindrical sponge-like (foamed resin) member that is to be overlayed on the rotation body base portion 113 such as to cover the side face of the rotation body base portion 113.

The material used to form the collector 14 may be, for example, at least any of a rubber, a resin and a non-woven fabric. When the collector 14 is made of a static-prone material, the static electricity generated in the collector 14 is utilized to enhance the efficiency of adhesion of the nucleic acids-containing sediment to the surface of the collector 14. When the collector 14 is made of a resin or a rubber having relatively high viscosity on the surface, the viscosity is utilized to enhance the efficiency of adhesion of the nucleic acids-containing sediment to the surface of the collector 14. The material used to form the collector 14 is desirably a material that is unlikely to change in properties by, for example, the water content in the soil when the collector 14 comes into contact with the surface of ground 20 to collect the nucleic acids-containing sediment. A viscous substance may further be placed on the surface of the collector 14 to enhance the efficiency of adhesion of the nucleic acids-containing sediment to the surface of the collector 14.

The thickness of the collector 14 provided as the sheet-like member is preferably to be small in terms of ensuring the flexibility for being wound around the rotation body base portion 113, while being preferably to be large in terms of ensuring the strength to withstand the operation of rotating the rotation body 112 for adhesion of the nucleic acids-containing sediment in the state that the collector 14 is wound around the rotation body base portion 113. The thickness of the collector 14 is appropriately set by taking into account the flexibility and the strength of the material used to form the collector 14. The thickness of the collector 14 may be, for example, not larger than 1 mm.

The collector 14 is provided with a mounting portion 15 at an end thereof to be mounted and fixed to the rotation body base portion 113 (as shown in Fig. 6(A)). Any of various configurations may be employed for the mounting portion 15, as long as the configuration enables the collector 14 to be attached to and detached from the rotation body base portion 113 and causes substantially no problem in the operation of rotating the rotation body 12 for adhesion of the nucleic acids-containing sediment in the state that the collector 14 is wound around the rotation body base portion 113. For example, the mounting portion 15 may include an adhesive to join respective ends of the collector 14 with each other. In another example, the mounting portion 15 may be a fixture element provided as a separate body from the collector 14 to cover the surface of the rotation body base portion 113. The mounting portion 15 may be a disposable member to be used only once. In the case where the mounting portion 15 is reused, the mounting portion 15 may be made of a material that allows for repetition of processes for suppressing the biological nucleic acids adhering to the mounting portion 15 in a previous use from being left or more specifically a process of washing the mounting portion 15 and a nucleic acid removal process using a chlorine bleaching agent, a nucleolytic agent or the like. The mounting portion 15 may be made of, for example, stainless steel or a resin. In the case where the mounting portion 15 is reused, with a view to facilitating the processes for suppressing the biological nucleic acids adhering to the mounting portion 15 in a previous use from being left, the mounting portion 15 preferably has a simple configuration that enables the mounting portion 15 to be readily soaked in a liquid and processed by the above processes. For example, the mounting portion 15 is desirably formed in a clip-like shape to clamp and fix the respective ends of the collector 14 along with the rotation body base portion 113.

The rotation body base portion 113 may be made of any material that has substantially no problem in use as the wheels for running of the apparatus for collecting biological nucleic acids 110. In the case where the rotation body base portion 113 is reused, the rotation body base portion 113 may be made of a material that allows for repetition of the above processes for suppressing the biological nucleic acids adhering to the rotation body base portion 113 in a previous use from being left. The rotation body base portion 113 may be made of, for example, stainless steel or a resin material such as silicone resin. The rotation body base portion 113 may, however, be a disposable member to be used only once.

The method of collecting biological nucleic acids by using the apparatus for collecting biological nucleic acids 110 described above provides the apparatus for collecting biological nucleic acids 110 at the process T110 of Fig. 1 and moves the apparatus for collecting biological nucleic acids 110 in the collection target area at the process T120, so as to cause the nucleic acids-containing sediment 22 to adhere to the surface of the collectors 14 included in the rotation bodies 112 (as shown in Fig. 6(B)). When it is determined at the process T130 that the operation for collection is completed, the method detaches the collectors 14 from the rotation body base portions 113 to collect the biological nucleic acids at the process T140. Fig. 6(C) illustrates the state of the rotation body 112a after detachment of the collector 14 and the state of the rotation body 112b prior to detachment of the collector 14.

The operation of collecting the biological nucleic acids from the collector 14 and extracting the biological nucleic acids may be performed by a method similar to the method illustrated in Fig. 5. Unlike the collector 14 made of a non-woven fabric or the like, the nucleic acids-containing sediment does not enter inside of the collector 14 made of a rubber or a resin. Accordingly, the method of Fig. 5 may wipe the surface of the collector 14 by using another member, for example, a similar member to that of the collector 30 of the first embodiment or a swab-like member, to collect the nucleic acids-containing sediment from the collector 14, in place of washing the collector 14 in the liquid, at the process T310. In this case, the biological nucleic acids may be extracted from the another member by the method shown in Fig. 4 or by the method shown in Fig. 5. It is preferable that the collector 14 is a disposable member to be used only once. In the case where the collector 14 that doe not allow the nucleic acids-containing sediment to enter inside of the collector 14 is wiped to collect the nucleic acids-containing sediment as described above, however, the collector 14 is made reusable by using the material that allows for repetition of the above processes for suppressing the biological nucleic acids from being left.

The configuration of the second embodiment using the apparatus for collecting biological nucleic acids 110 provided with the rotation bodies to collect the nucleic acids-containing sediment present on the surface of ground has similar advantageous effects to those of the configuration of the first embodiment.

### C. Third Embodiment

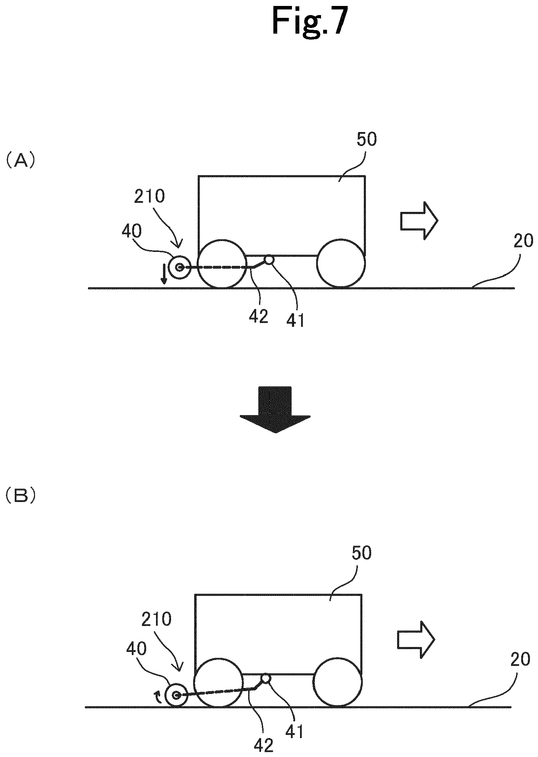

Fig. 7 is explanatory diagrams illustrating an operation of collecting the nucleic acids-containing sediment on the surface of ground by using an apparatus for collecting biological nucleic acids 210 according to a third embodiment. The apparatus for collecting biological nucleic acids 210 of the third embodiment is attached in use to a running vehicle 50 that is provided outside of the apparatus for collecting biological nucleic acids 210 and that is configured to autonomously move. The apparatus for collecting biological nucleic acids 210 is provided with a rotation body 40 that rotates accompanied with moving of the running vehicle 50 while coming into contact with the surface of ground 20 and thereby causes the nucleic acids-containing sediment, which is present on the surface of ground 20 and which includes biological nucleic acids, to adhere to a surface thereof.

The running vehicle 50 is a land moving vehicle like the apparatus for collecting biological nucleic acids 10 of the first embodiment or the apparatus for collecting biological nucleic acids of the second embodiment and may employ any of various configurations as described in the first embodiment and the second embodiment. In the first embodiment and the second embodiment, the wheels for running of the moving vehicle are used as the rotation bodies to collect the nucleic acids-containing sediment. The rotation body 40 provided in the apparatus for collecting biological nucleic acids 210 of the third embodiment is, on the other hand, a separate member from wheels for running of the running vehicle 50 which the apparatus for collecting biological nucleic acids 210 is attached to.

The apparatus for collecting biological nucleic acids 210 shown in Fig. 7 is provided with a connection member 42 configured to connect the rotation body 40 with the running vehicle 50, in addition to the rotation body 40 operated to cause the nucleic acids-containing sediment present on the surface of ground 20 to adhere to the surface thereof. The connection member 42 of this embodiment is configured by an arm-like member. The connection member 42 has one end that is attached to a rotating shaft 41 provided in the running vehicle 50 such as to rotate about the rotating shaft 41. The rotation body 40 is provided at the other end of the connection member 42. The connection member 42 is not limited to the arm-like member shown in Fig. 7 but may have any configuration that causes the rotation body 40 to be connected with the running vehicle 50.

When the apparatus for collecting biological nucleic acids 210 attached to the running vehicle 50 is not used for collection of the nucleic acids-containing sediment, the connection member 42 may be engaged with the running vehicle 50 by using, for example, a non-illustrated engagement element to keep the rotation body 40 at a position away from the surface of ground 20. On the start of collection of the nucleic acids-containing sediment by using the apparatus for collecting biological nucleic acids 210, the engagement by the engagement element may be released to cause the rotation body 40 to fall on the surface of ground 20. In Fig. 7(A), a downward arrow indicates the state that the rotation body 40 falls down, accompanied with rotation of the connection member 42 about the rotating shaft 41. The rotation body 40 then rotates accompanied with moving of the running vehicle 50 while coming into contact with the surface of ground 40 and, as a result, causes the nucleic acids-containing sediment present on the surface of ground 20 to adhere to the surface of the rotation body 40. A different mechanism from that of Fig. 7 may be employed as the switching mechanism to switch over the state of the rotation body 40 between the state that the rotation body 40 comes into contact with the surface of ground 20 and the state that the rotation body 40 does not come into contact with the surface of ground 20 before and after the start of the operation for collection of the nucleic acids-containing sediment.

Providing the switching mechanism facilitates, for example, the switching operation of keeping the rotation body 40 at the position away from the surface of ground 20 when the running vehicle 50 runs in a place where the operation for collection of the nucleic acids-containing sediment by using the rotation body 40 is not appropriate, while bringing the rotation body 40 into contact with the surface of ground 20 when the running vehicle 50 runs in a place where the operation for collection is appropriate. The apparatus for collecting biological nucleic acids 210 may be attached to the running vehicle 50 every time the operation for collection of the nucleic acids-containing sediment is performed.

The rotation body 40 provided in the apparatus for collecting biological nucleic acids 210 is attached on a rear side in a moving direction of the running vehicle 50 in the illustrated example of Fig. 7 but may be attached on a front side in the moving direction. The rotation body 40 may also be attached on a lateral side of the running vehicle 50 to be protruded in a width direction of the running vehicle 50. This configuration, however, increases the width of the running vehicle 50 and is thus likely to have some limitations in the location where the running vehicle 50 enters. This configuration is also likely to lower the stability of running of the running vehicle 50 and the stability of rotation of the rotation body 40. This is thus likely to lower the efficiency of collection of the nucleic acids-containing sediment. It is accordingly preferable that the connection member 42 attaches the rotation body 40 to be protruded on the front side or on the rear side of the running vehicle 50 or attaches the rotation body 40 such as to be placed inside of an outer periphery of the running vehicle 50 that is viewed in projection in the vertical direction.

In the apparatus for collecting biological nucleic acids 210, the force of pressing the rotation body 40 against the surface of ground 20 may be obtained by the dead weight of the rotation body 40, or the connection member 42 may be configured to apply the force of pressing the rotation body 40 against the surface of ground 20.

According to one modification, the rotation body 40 in the apparatus for collecting biological nucleic acids 210 may have a configuration similar to the configuration of the rotation body 12 of the first embodiment that causes the nucleic acids-containing sediment to directly adhere to the surface of the rotation body 40. The surface of the rotation body 40 is then wiped by using another member, for example, a similar member to that of the collector 30 of the first embodiment or a swab-like member, and the nucleic acids-containing sediment is collected from the another member. According to another modification, the apparatus for collecting biological nucleic acids 210 may be provided with a collector similar to the collector 30 of the first embodiment to collect the nucleic acids-containing sediment from the surface of the rotation body 40. According to another modification, the rotation body 40 may be configured to include a rotation body base portion 113 and a collector 14, like the rotation body 112 of the second embodiment. The nucleic acids-containing sediment adhering to the collector 14 accompanied with running of the running vehicle 50 may be collected by detachment of the collector 14 from the rotation body 40. In these modifications, the materials used to form the rotation body 40, the collector 30, the rotation body base portion 113 and the collector 14 may be selected as described in the first embodiment and the second embodiment. The nucleic acids-containing sediment may be collected from the member corresponding to the collector 30 or the collector 14 described above, and the biological nucleic acids may be extracted by the method shown in Fig. 4 or by the method shown in Fig. 5.

The configuration of the third embodiment using the apparatus for collecting biological nucleic acids 210 provided with the rotation body to collect the nucleic acids-containing sediment present on the surface of ground has similar advantageous effects to those of the configuration of the first embodiment.

### Examples

Biological nucleic acids were collected from the environment according to the method of collecting biological nucleic acids of the present disclosure, and the species of organisms were identified from the obtained biological nucleic acids. In particular, two 20 kilometer areas in Aichi Prefecture were set as collection target areas. The biological nucleic acids were collected in each of the collection target areas in April, 2021 and in June, 2021. The apparatus for collecting biological nucleic acids 10 shown in Fig. 2 and provided with the rotation bodies 12 having the rubber surface were moved in each of the above 20 kilometer areas to cause the nucleic acids-containing sediment to adhere to the surface of the rotation bodies 12. The nucleic acids-containing sediment adhering to the rotation bodies 12 were collected by pressing a collector member made of chemical fibers against the rotation bodies 12, and biological nucleic acids were extracted from the collector member according to the method of Fig. 4. At the process T140, DNA extraction was performed from the collector member by using a commercially available DNA extraction kit for soil samples (NucleoSpin (registered trademark) Soil manufactured by Takara Bio Inc.)

Each nucleic acid extract obtained by using the above kit was used as an environmental DNA sample in the collection target area, and a 12SrRNA sequence was amplified by the PCR method using predetermined primers (existing bird species universal primers). A Kapa Hifi PCR kit (manufactured by Kapa Biosystems, Inc.) was used for the amplification, and the PCR was repeated four times. Another PCR was performed for addition of an index for analysis of the sequence, and the PCR product was subjected to beads purification. A sequence was then obtained by a next generation sequencer. The sequencer used was Illumina iSeq (registered trademark) 100 (manufactured by Illumina, Inc.)

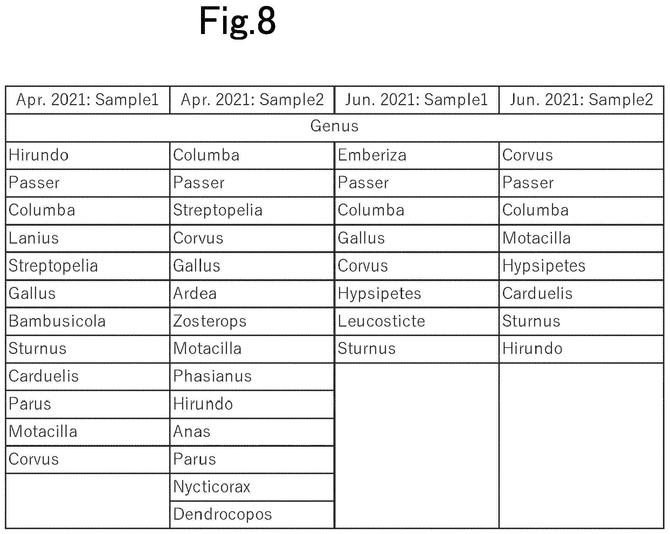

Fig. 8 is an explanatory diagram illustrating the results of analysis of the above environmental DNA samples. The species of organisms were extracted from the obtained sequences by MiFish Pipeline as a pipeline for DNA analysis and were identified by BLAST homology search. The list of Fig. 8 shows only the species of organisms having the homology of not lower than 97% based on the database. Fig. 8 shows the results of the identification of the species of organisms in the genus level. As shown in Fig. 8, it has been confirmed that a wide variety of terrestrial bird species live or stayed in these collection target areas by the method of collecting biological nucleic acids according to the present disclosure. The two collection target areas that are relatively near to each other show the similar results that a wider variety of bird species were found in the spring (April) than in the summer (June).

The present disclosure is not limited to the above embodiments described above but may be implemented in a variety of configurations without departing from the subject matter or the scope of the present disclosure. For example, the technical features of the embodiments corresponding to the technical features of the respective aspects described in Summary may be appropriately changed, altered, replaced or combined, in order to solve part or the entirety of the problems described above or in order to achieve part or the entirety of the advantageous effects described above. The technical features may be appropriately omitted unless the technical features are mentioned as essential in the description hereof.

It is explicitly stated that all features disclosed in the description and/or the claims are intended to be disclosed separately and independently from each other for the purpose of original disclosure as well as for the purpose of restricting the claimed invention independent of the composition of the features in the embodiments and/or the claims. It is explicitly stated that all value ranges or indications of groups of entities disclose every possible intermediate value or intermediate entity for the purpose of original disclosure as well as for the purpose of restricting the claimed invention, in particular as limits of value ranges.

## Claims

1. A biological nucleic acid collection method of collecting biological nucleic acids from an environment, the biological nucleic acid collection method comprising:
setting a collection target area that is a subject for collecting the biological nucleic acids in the environment;
rotating a rotation body (12; 40; 112) with a surface of the rotation body (12; 40; 112) coming into contact with surface of ground in the collection target area, so as to cause nucleic acids-containing sediment including the biological nucleic acids present on the surface of ground to adhere to the surface of the rotation body (12; 40; 112); and
collecting the nucleic acids-containing sediment from the rotation body (12; 40; 112).

2. The biological nucleic acid collection method according to claim 1, further comprising:
extracting nucleic acids from the collected nucleic acids-containing sediment.

3. A biological nucleic acid collection apparatus (10; 110; 210) configured to collect biological nucleic acids from an environment, the biological nucleic acid collection apparatus (10; 110; 210) comprising:
a rotation body (12; 40; 112) configured to rotate accompanied with moving of the biological nucleic acid collection apparatus (10; 110; 210) while coming into contact with surface of ground, so as to cause nucleic acids-containing sediment including biological nucleic acids present on the surface of ground to adhere to a surface of the rotation body (12; 40; 112); and
a collector (14; 30) configured to collect the nucleic acids-containing sediment adhering to the surface of the rotation body (12; 40; 112).

4. The biological nucleic acid collection apparatus (10) according to claim 3,
wherein the collector (30) comes into contact with the surface of the rotation body (12) to collect the nucleic acids-containing sediment adhering to the surface of the rotation body (12).

5. The biological nucleic acid collection apparatus (10) according to claim 4,
wherein the collector (30) comprises at least one of a rotational member, a brush-like member and a duster-like member.

6. The biological nucleic acid collection apparatus (110) according to claim 3,
wherein the collector (14) is provided to be attached to and detached from the rotation body (112), such as to form the surface of the rotation body (112) that comes into contact with the surface of ground.

7. The biological nucleic acid collection apparatus (110) according to claim 6,
wherein the collector (14) is formed in a sheet-like shape.

8. The biological nucleic acid collection apparatus (110) according to either claim 6 or claim 7,
wherein the collector (14) is made of at least one of a rubber, a resin, and a non-woven fabric.

9. The biological nucleic acid collection apparatus (10; 110) according to any one of claims 3 to 8,
wherein a ratio of an area of the rotation body (12; 112) to a total area of the entire biological nucleic acid collection apparatus (10; 110) that is viewed in projection in a vertical direction is not less than 8%.

10. The biological nucleic acid collection apparatus (210) according to any one of claims 3 to 9, further comprising:
a connection member (42) configured to connect the rotation body (40) with an external running vehicle configured to autonomously move.
